# EUROPEAN PATENT APPLICATION

(11) **EP 1 454 544 A2**
(43) Date of publication of application: **08.09.2004**
(21) Application number: 04004274.9
(22) Date of filing: 25.02.2004
(51) Int. Cl.: A43B 3/30, A43B 7/26, A43B 17/00

(54) **Set of innersoles for baby shoes**

(30) Priority: 25.02.2003 JP 2003048165
(71) Applicant: Aprica Kassai Kabushikikaisha, Osaka-shi, Osaka 542-0082 (JP)
(72) Inventor: Kassai, Kenzou, Osaka-shi Osaka 542-0083 (JP); Suzuki, Sachiyo, Chuo-ku Osaka-shi Osaka 542-0082 (JP)
(74) Representative: Hofer, Dorothea, Dipl.-Phys.

(57) **Abstract**

A set of inner soles for baby shoes comprises a first inner sole (10) and a second inner sole (30). The first inner sole (10) has a partition wall (11) rising between the first and second toes of a baby foot. The second inner sole (30) has an inverted T-shaped projection part (31) comprising a longitudinal projection part (32) projecting a little and extending between the first and second toes and a lateral projection part (33) extending under bases of the first and second toes.

## Description

### BACKGROUND OF THE INVENTION

### Field of the Invention

The present invention relates to an insole for baby shoes and more particularly, it relates to an insole which promotes formation of the arch of a foot of a baby.

### Description of the Background Art

Baby shoes are used from an early stage of walking, which is for the purpose of protecting feet of a baby.

It is said that a foot of a child starts to grow from about one year old when a baby starts to walk until six or seven years old. Especially, it is important to develop the foot in a healthy manner in a relatively early stage of growth.

One typical example of healthy growth of the foot includes the growth of the arch of the foot. The arch of the foot absorbs an impact at the time of walking and plays an important role in protecting the brain through the back bone. It is said that it is important to walk while exercising each toe in such a manner that the foot may catch the ground, in order to form the arch of the foot. Such walking manner is more easily implemented when the baby walks barefoot.

In the meantime, shoes are necessary for protecting the foot. When the baby puts on shoes, the movement of each toe of the foot is hindered and it is difficult to exercise the foot in catching the ground. As a result, the healthy growth of the foot could be hindered.

The same applicant as in the present application disclosed an insole for baby shoes improved so as to promote the growth of the foot of the baby in Japanese Unexamined Patent Publication No. 2001-157601.

The insole disclosed in Japanese Unexamined Patent Publication No. 2001-157601 is used when the baby puts on shoes barefoot, especially. Therefore, when the baby puts on shoes wearing socks, the insole disclosed in Japanese Unexamined Patent Publication No. 2001-157601 cannot be used. In an everyday life of the baby, however, there are mixed cases where the baby puts on shoes wearing socks and not wearing socks.

### SUMMARY OF THE INVENTION

It is an object of the present invention to provide an insole set for baby shoes which can effectively promote the formation of the arch of a foot, in both cases where the baby puts on shoes barefoot and where puts on shoes wearing socks.

An insole set for baby shoes comprises a first insole and a second insole. The first insole has a partition wall rising between the first and second toes of a baby foot. The second insole has an inverted T-shaped projection part comprising a vertical projection part projecting a little and extending between the first and second toes and a lateral projection part extending under bases of the first and second toes.

Preferably, the insole set further comprises a third insole having a flat configuration with no projection.

A height of the partition wall of the first insole is preferably the same as that of the toes of the baby foot or higher than them. A height of the vertical and lateral projections of the second insole is preferably 5mm or less.

As a typical usage manner, the first insole is for a case the baby is barefoot, the second insole is for a case the baby wears socks and the third insole is for adjusting the height.

According to one embodiment, the insole set further comprises a fourth insole and a fifth insole. The fourth insole has a partition wall rising between the first and second toes of the baby foot and a projection for an arch of the foot abutting on the arch of the baby foot. The fifth insole has an inverted T-shaped projection part comprising a vertical projection part projecting a little and extending between the first and second toes and a lateral projection part extending under bases of the first and second toes and a projection for an arch of the foot abutting on the arch of the baby foot.

An operational effect of each component will be described in the section of the description of the embodiment.

### BRIEF DESCRIPTION OF THE DRAWINGS

Fig. 1 is a view showing a track in a shift of the center of gravity of a foot of an adult;
Fig. 2 is a view in which a baby foot is divided into a front part and a rear part in order to analyze a pressure distribution;
Fig. 3 is views showing variations in load of a right foot with the passage of time;
Fig. 4 is views showing tracks in shifts of the center of gravity in soles:
Fig. 5 is views showing tracks in the shift of the center of gravity in right and left feet;
Fig. 6 is a perspective view showing an example of an insole;
Fig. 7 is a plan view showing the insole in Fig. 6;
Fig. 8 is a sectional view taken along line 8-8 in Fig. 7;
Fig. 9 is a plan view showing another example of an insole;
Fig. 10 is a sectional view taken along line 10-10 in Fig. 9;
Fig. 11 is a plan view showing still another example of an insole;
Fig. 12 is a plan view showing the insole in Fig. 11;
Fig. 13 is a sectional view taken along line 13-13 in Fig. 12;
Fig. 14 is a plan view showing still another example of an insole;
Fig. 15 is a sectional view taken along line 15-15 in Fig. 14;
Fig. 16 is a perspective view showing still another example of an insole;
Fig. 17 is a perspective view showing still another example of an insole;
Fig. 18 is a sectional view in which a partition wall of the insole shown in Fig. 17 is cut in the longitudinal direction;
Fig. 19 is a sectional view in which a partition wall of the insole shown in Fig. 17 is cut in the width direction;
Fig. 20 is a view showing an example of an insole set;
Fig. 21 is a view showing another example of an insole set:
Fig. 22 is a view showing still another example of an insole set:
Fig. 23 is a view showing still another example of an insole set:
Fig. 24 is a view showing still another example of an insole set:
Fig. 25 is a view showing still another example of an insole set: and
Fig. 26 is a view showing still another example of an insole set:

### DESCRIPTION OF THE PREFERRED EMBODIMENTS

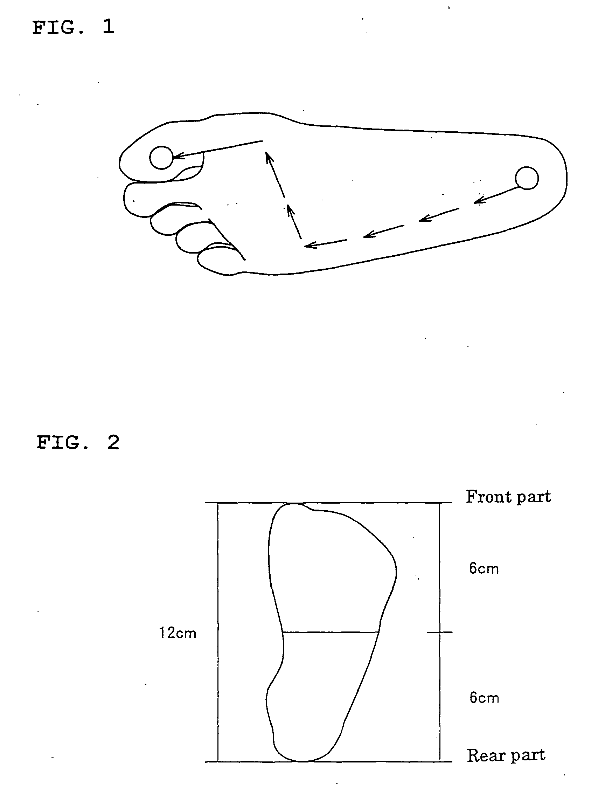

Fig. 1 shows a sole of a foot of an adult in which the arch of the foot is formed. In Fig. 1, arrows trace a shift of weight while walking. Attending to a shift of the center of gravity while the adult walks, the center of gravity reaches the ground first at a heel and then shifts to the outer side of the foot and shifts from a head of the fifth metatarsal bone to a head of the first metatarsal bone and comes off from a tip end of the big toe (first toe).

In a case of a foot of a baby who just began walking, since the arch of the foot is not formed, a shift of the center of gravity is completely different. The inventor of the present invention analyzed walking of the baby by measuring a pressure distribution of the sole. More specifically, it was previously assumed that there was a difference in pressure applied to the sole between a baby who just begun walking and a baby who got used to walking, and measurement was performed using a pressure distribution mat in order to figure out a state and the center of gravity of a load applied to the sole while walking.

A variation in load and a shift of the center of gravity are compared by walking the baby being tested freely so as to respond to calls on the pressure distribution mat when the baby is thirteen, fourteen and fifteen months old. According to the pressure distribution mat used in the experiment, sensor resolution is 1 [cm²], a size of a sensor part is 48cm x 44cm and normal force can be output as pressure information. A measurement range of the sensor is 1.96 to 19.6 [kPa] and precision is ± 10%. Two of the mats were connected such that an effective measurement region might be 96cm x 44cm, and then the measurement was performed at a sampling frequency 100 [Hz].

One step by a right foot the standing time of which is almost the same is selected from each of results measured when the baby is thirteen, fourteen and fifteen months old and compared with each other. Since the sole of the baby is immature and flat, the load distribution is divided to a front part and a rear part at the center of the sole in the longitudinal direction as shown in Fig. 2.

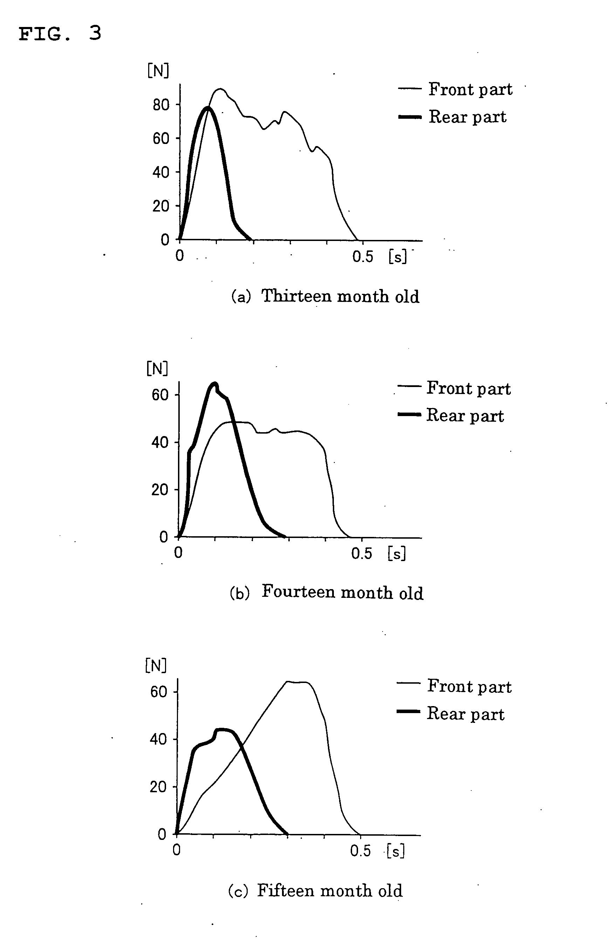

Fig. 3 shows variations in load in the right sole with the passage of time. According to a time when the load of the rear part becomes zero [N] at the age of thirteen, fourteen and fifteen months old, respectively, it is 0.19 [s] at the age of thirteen months old, 0.29 [s] at the age of fourteen months old and 0.31 [s] at the age of fifteen months old, and it is seen that the loaded time to the rear part is gradually increased with the growth. This means that the walking makes progress from immature walking in which the sole reaches the ground as almost a whole and then the heel comes off, to mature walking in which the heel reaches the ground first and the load gradually shifts to the front part.

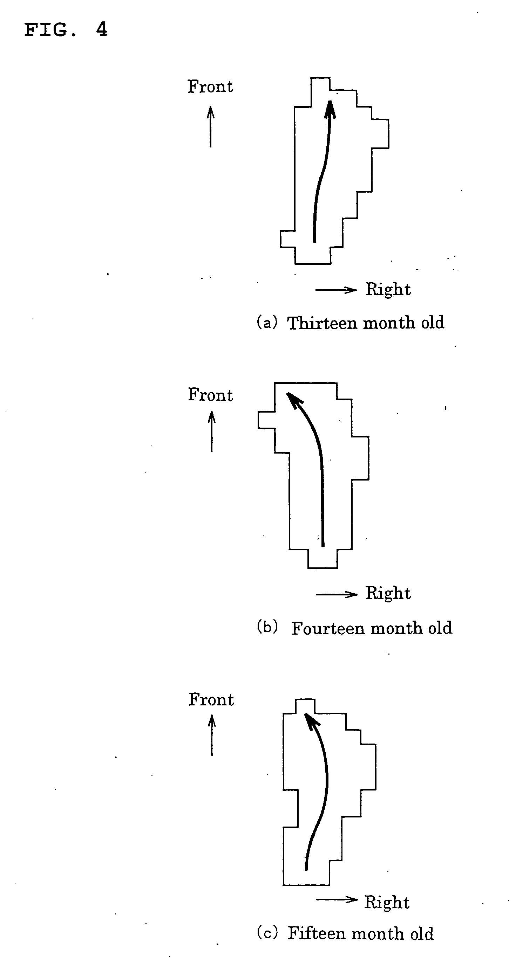

Fig. 4 shows tracks in shifts of the center of gravity of the soles. While the center of gravity linearly shifts from the heel to the toe at the age of thirteen months old, the center of gravity shifts to the toe drawing an arc at the age of fourteen and fifteen months old. In other words, it is seen that a sense of stability is brought about when the center of gravity moves in the width direction of the sole, which means the walking has made progress.

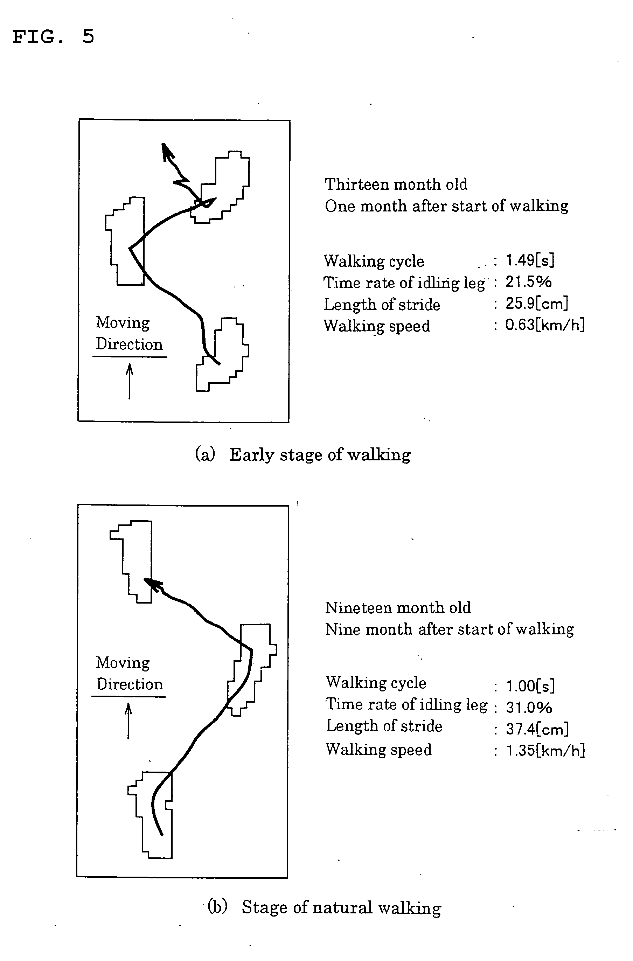

The inventor of the present invention further investigated tracks in the shifts of the center of gravity of right and left feet of the baby. Fig. 5 shows tracks of the shifts of the center of gravity, time factors and distance factors of a thirteen-month-old baby (one month has passed after the start of walking) as an example when the walking is just started, and a nineteen-month-old baby (nine months has passed after the start of walking) as an example when the walking becomes natural.

As can be clear from comparison between Fig. 5(a) and 5(b), the center of gravity shifts from the center of one foot to the other foot in the early stage of walking. This is because at the time of standing on one foot, the next foot reaches the ground before the center of gravity shifts to the front part of the foot and the center of gravity shifts in a state in which both feet are on the ground. In other words, the center of gravity does not effectively shift.

In the meantime, when the walking becomes natural, the center of gravity shifts from the heel to the front part of the foot and then shifts to the heel of the next foot. In other words, the center of gravity shifts from the heel to the front part of the sole at the time of standing on one foot, and then the next foot reaches the ground. This is thought to be a sign of movement in which the front part of the foot kicks the ground.

According to a time rate of an idling leg which shows a ratio of time for standing on one foot in one walking cycle, while it is 21.5% in the stage of immature walking, it is increased to 31.0% in the stage of natural walking, which means that stability is increased at the time of standing on one foot. According to a length of stride, while it is 25.9cm in the early stage of walking, it is also increased also to 37.4cm in the stage of natural walking, and similarly, walking speed is also increased from 0.63km/h to 1.35km/h. In view of the above, it is understood that walking efficiency is improved.

As a result of the above measurement, it is recognized that as the baby gets used to walking, the stability in walking and the walking efficiency are improved and the walking is improved.

In view of the walking characteristics of the baby described above, the present invention was made to provide an insole set and an insole which can appropriately promote the formation of the arch of the foot depending on the stages of growth.

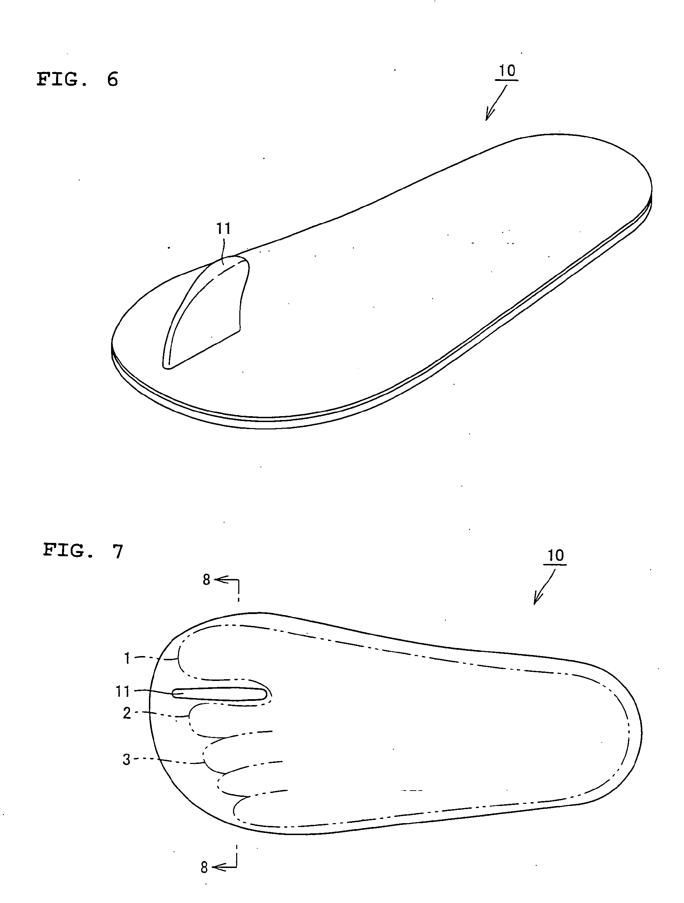

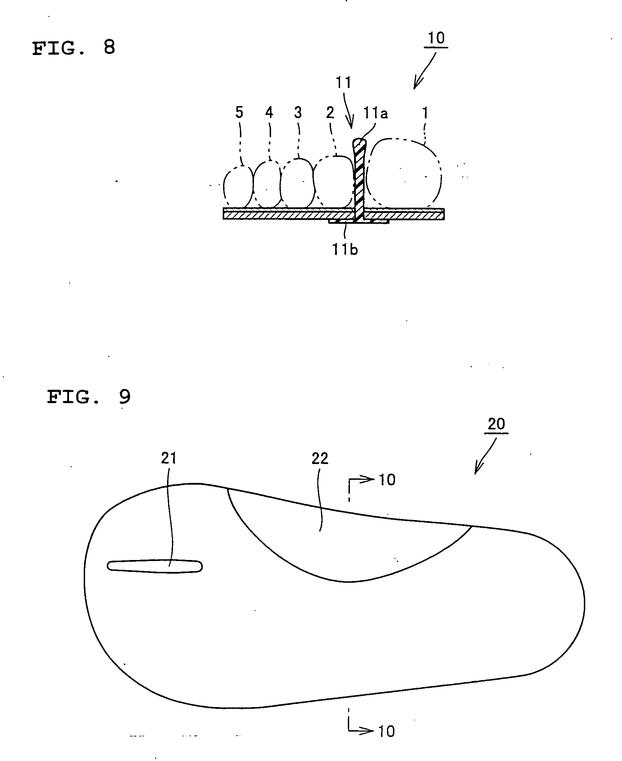

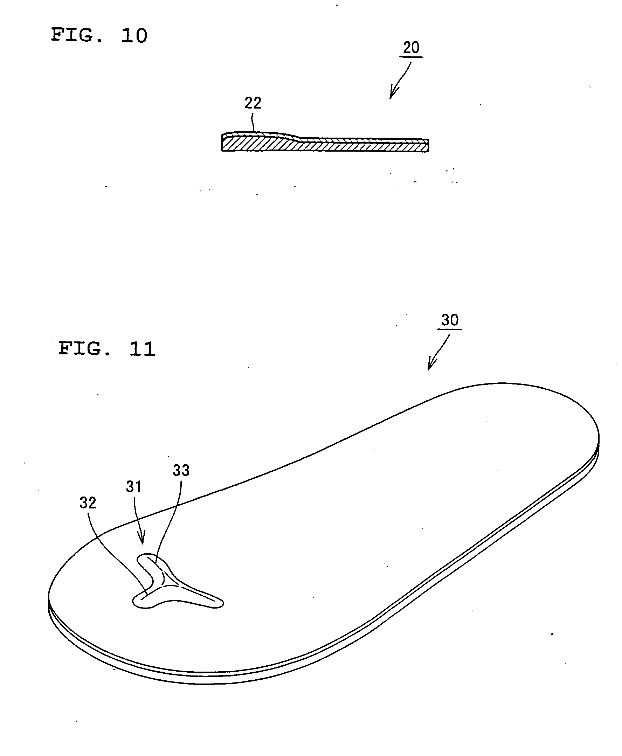

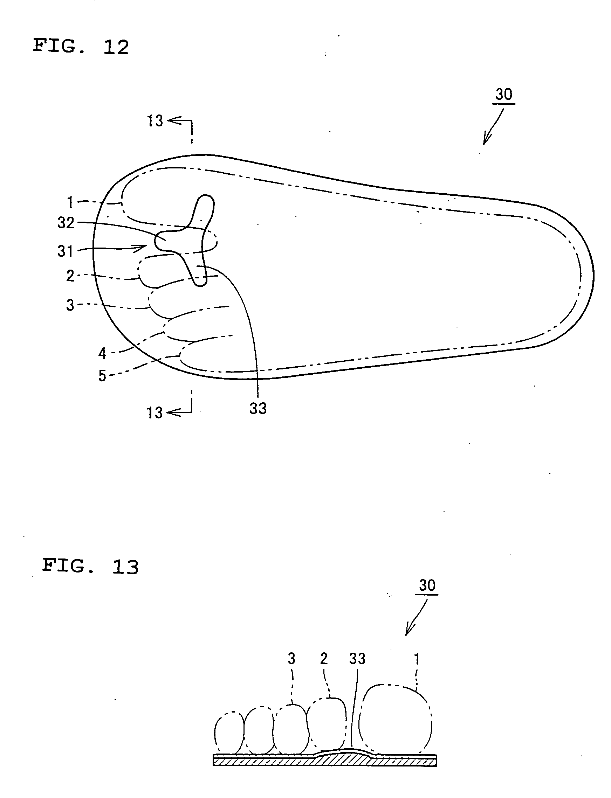

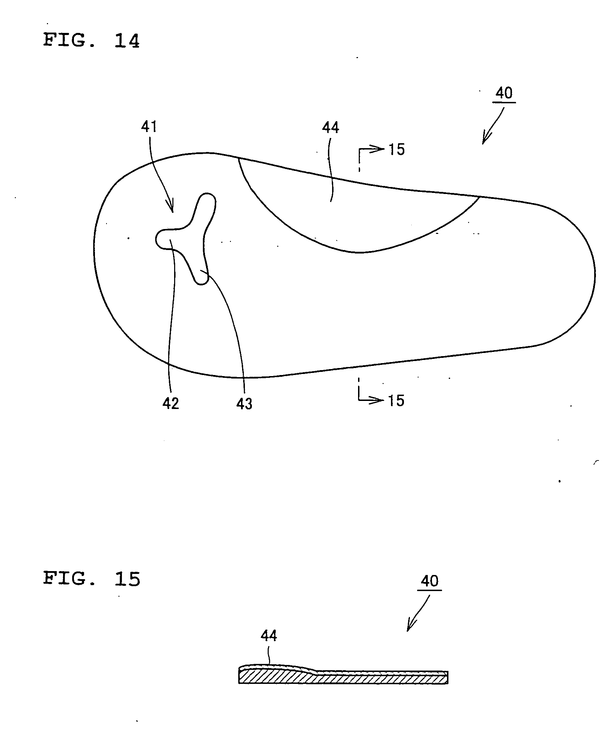

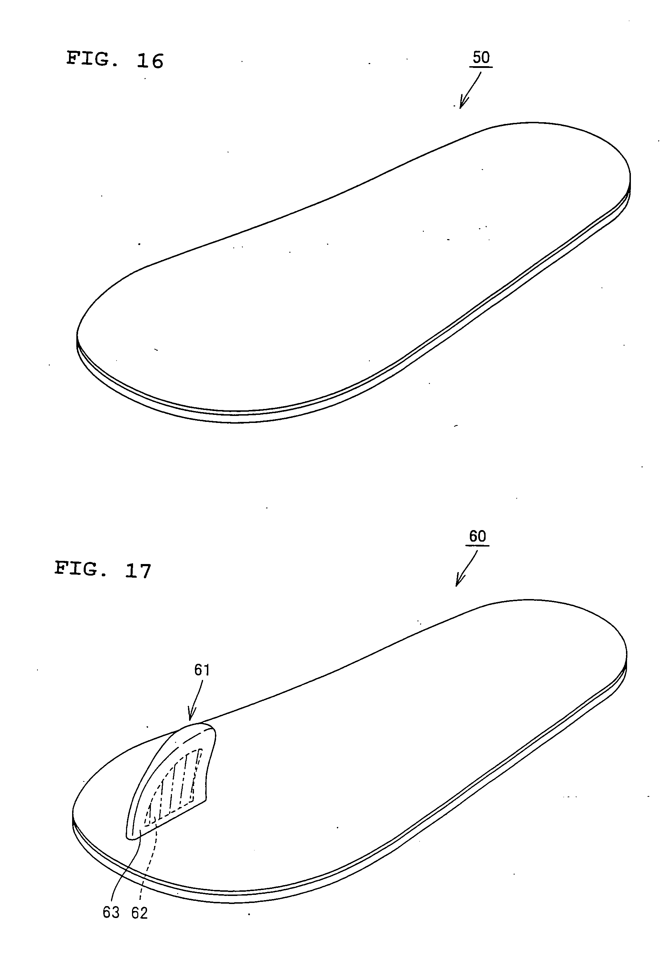

The insole set comprises a first insole 10 shown in Figs. 6 to 8, a second insole 20 shown in Figs. 9 and 10, a third insole 30 shown in Figs. 11 to 13, a fourth insole 40 shown in Figs. 14 and 15, and fifth insole 50 shown in Fig. 16.

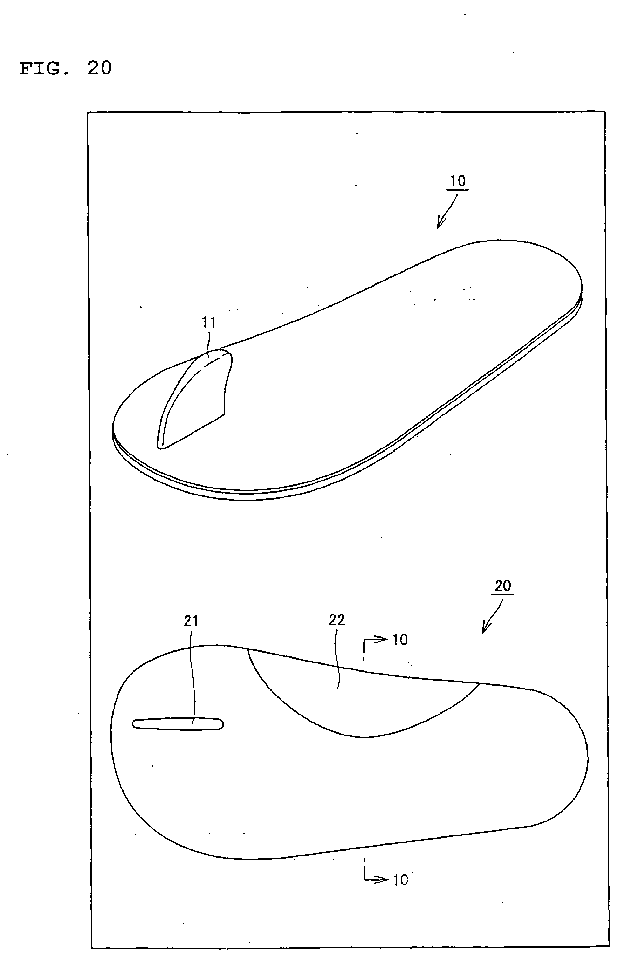

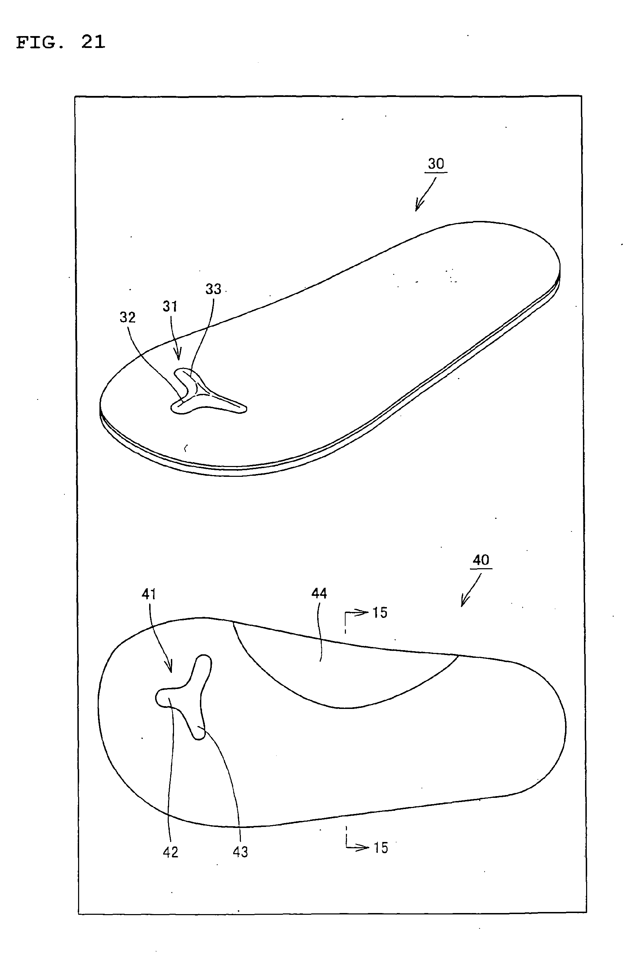

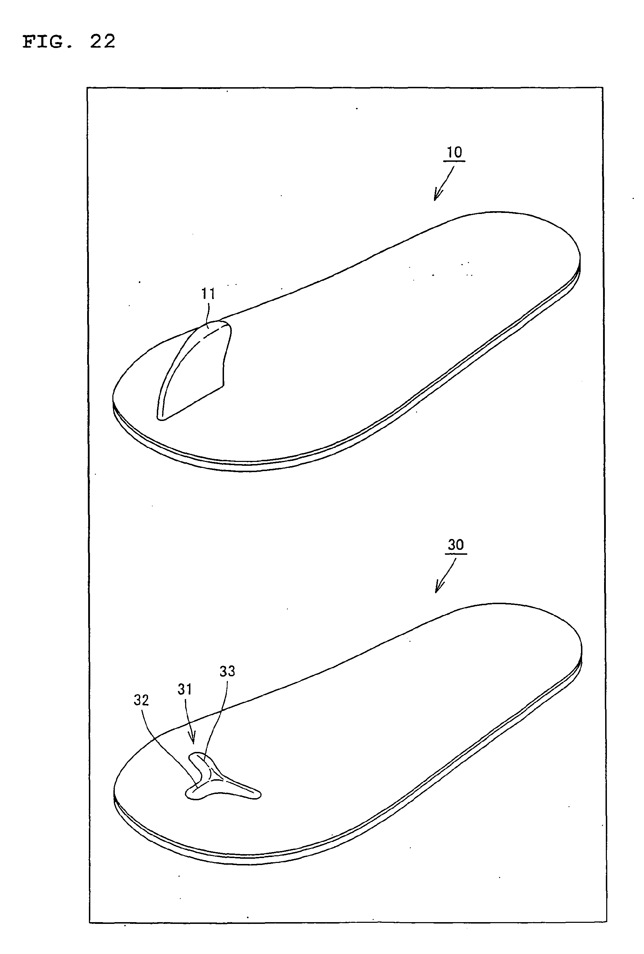

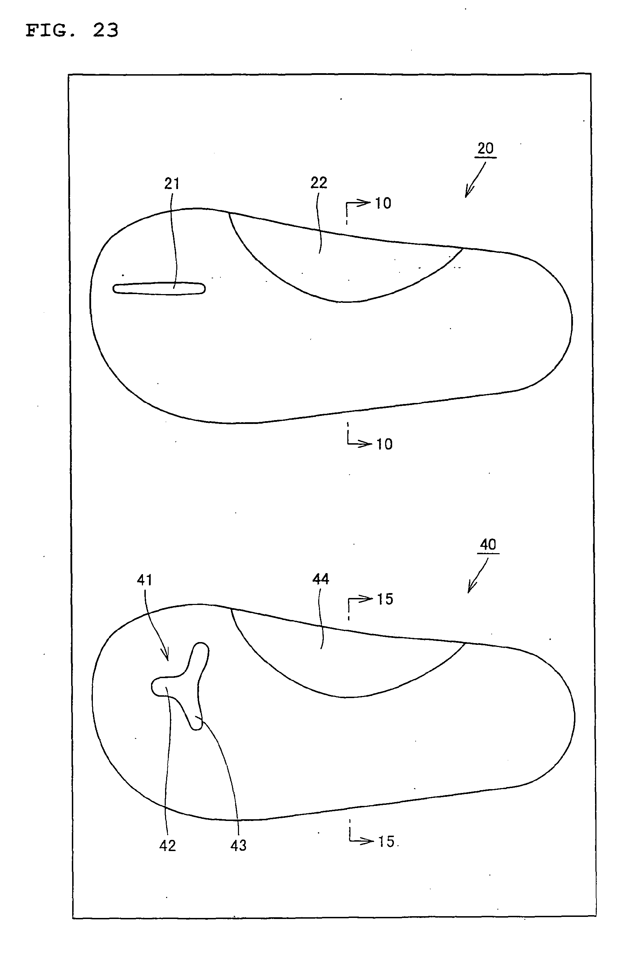

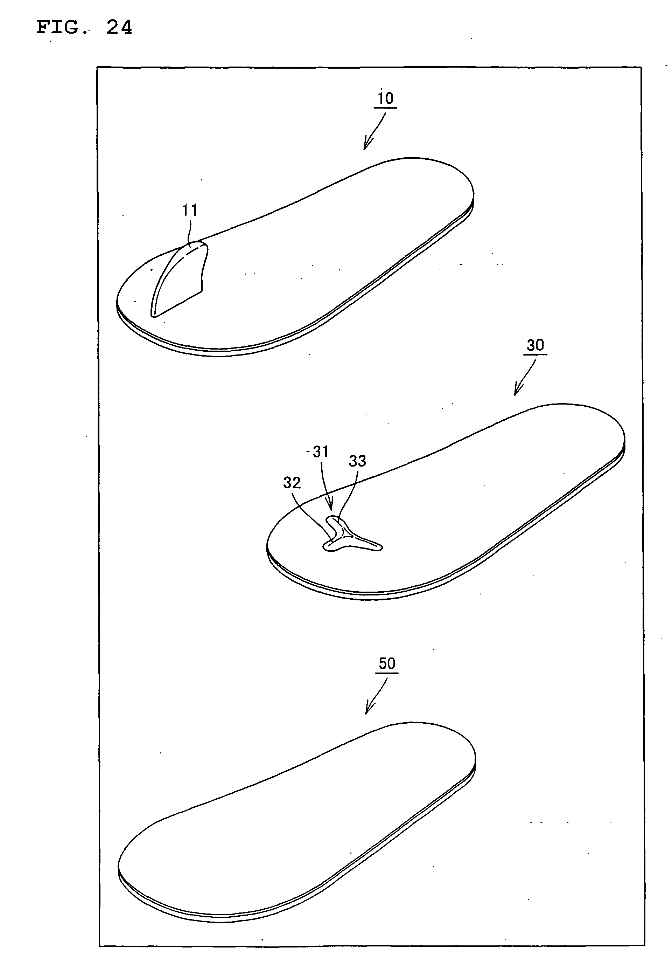

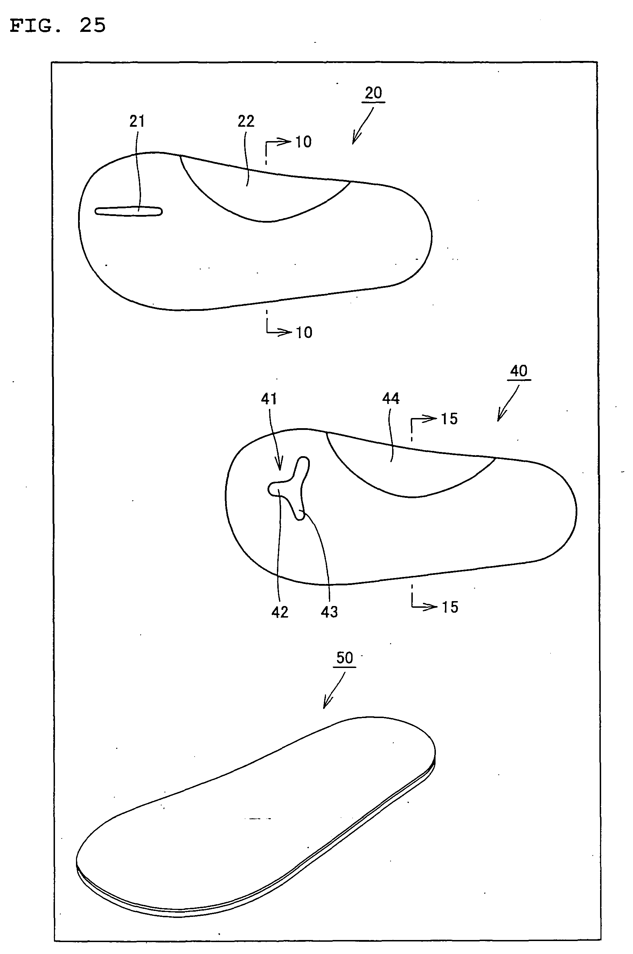

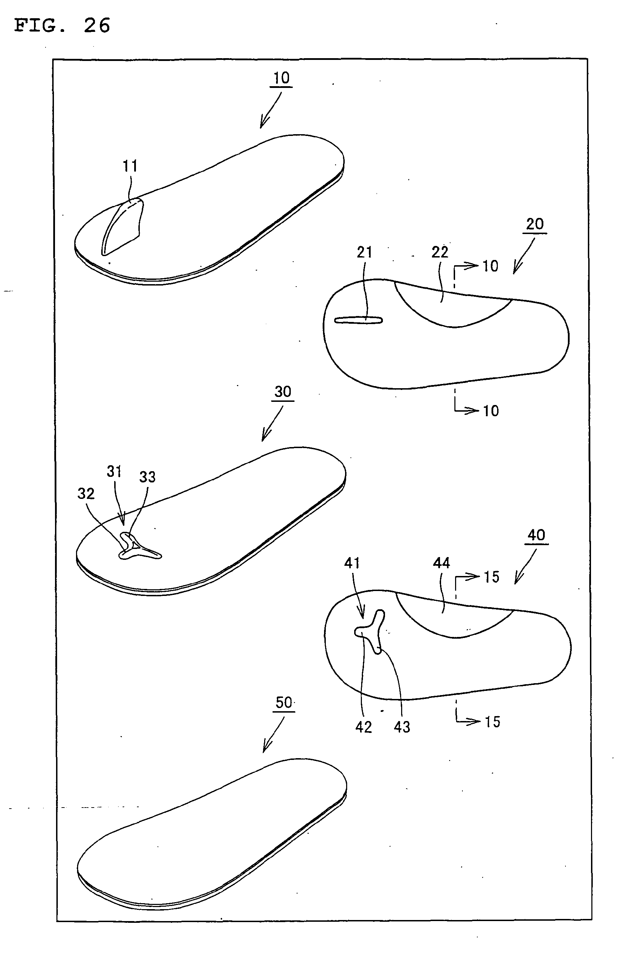

Although the insole set is made by combining various kinds of insoles, there are various kinds of combinations depending on its purpose. For example, the insole set is composed of the following combinations of the insoles.
A. The first insole 10 + the second insole 20 (Fig. 20)
B. The third insole 30 + the fourth insole 40 (Fig. 21)
C. The first insole 10 + the third insole 30 (Fig. 22)
D. The second insole 20 + the fourth insole 40 (Fig. 23)
E. The first insole 10 + the third insole 30 + the fifth insole 50 (Fig. 24)
F. The second insole 20 + the fourth insole 40 + the fifth insole 50 (Fig. 25)
G. The first insole 10 + the second insole 20 + the third insole 30 + the fourth insole 40 + fifth insole 50 (Fig. 26)

Each of the first insole 10 shown in Fig. 6 and the third insole 30 shown in Figs. 10 and 11 has a projection to be sandwiched between the first and second toes of the foot of the baby and a flat configuration except the projection. The insoles 10 and 30 are suitable for the baby who just begun walking. The walking manner of the baby who just begun walking is flat walking in which the sole reaches the ground almost all together and the center of gravity shifts linearly and comes off at the big toe (the first toe).

Since the baby pinches the projection by the first and second toes, the existence of the projection promotes the formation of the arch of the foot of the baby. Since the baby toddles with the flat soles in the early stage of walking, the region other than the projection is preferably flat. For example, if there is a projection in the insole at the part of the arch of foot, balance between right and left is lost and the walking of the baby becomes unstable.

The first and third insoles 10 and 30 are described further in detail.

The first insole 10 comprises a partition wall 11 rising between the first and second toes 1 and 2 of the foot of the baby. The first insole 10 is used when the baby puts on shoes barefoot. As shown in Fig. 8, the partition wall 11 is a flat plate-shaped member extending between the first and second toes 1 and 2 and an upper end 11a thereof is thickened a little. In addition, at its base end, a base end plate 11b is formed on a back surface of the insole. The region except the partition wall 11 is flat in order to be better suited for the toddling baby.

As shown in Fig. 8, a height of the partition wall 11 is the same as that of the toes or it is higher than the toes. Since the baby pinches the partition wall 11 by the first and second toes when walks, the arch of the foot can be well formed.

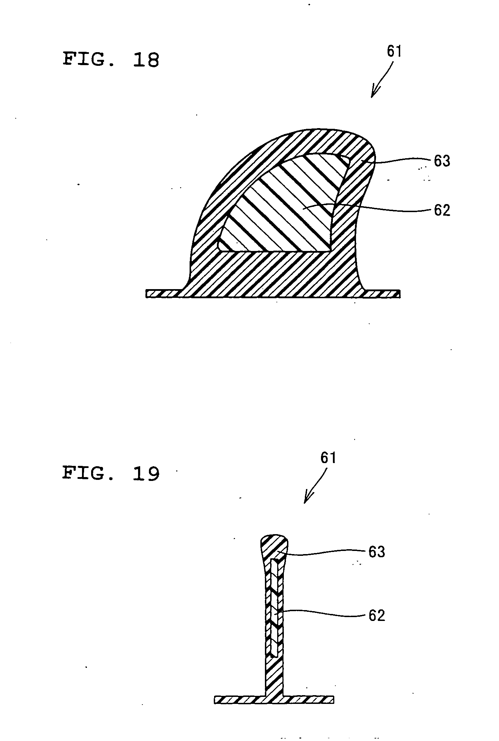

According to the sixth insole 60 shown in Figs. 17 to 19, an improvement has been added to the first insole 10. The sixth insole 60 has a partition wall 61 rising between the first and second toes. As shown in the drawings, the partition wall 61 has a hard material part 62 which is relatively hard and flat, and a soft material part 63 which is relatively soft and wraps around the hard material part 62. The hard material part 62 is formed of a hard silicon rubber, for example and the soft material part 63 is formed of a soft silicon rubber, for example.

The partition wall 61 is directly in contact with the very sensitive and weak skin of the baby. According to the sixth insole 60, since the soft material part 63 comes in contact with the skin of the baby, the skin is not damaged. Meanwhile, the hard material part 62 which is hard inside stably keeps the partition wall 61 uprising.

The third insole 30 shown in Figs. 11 to 13 comprises an inverted T-shaped projection part 31 having a vertical projection part 32 projecting a little and extending between the first and second toes 1 and 2, and a lateral projection part 33 extending to the bases of the first and second toes 1 and 2. Since a height of the projection part in the third insole 30 is low, the third insole 30 is suitable for being used when the baby puts on shoes with socks. In addition, it is needless to say that the third insole 30 can be used when the baby puts on shoes barefoot.

Considering the case where the foot is put on the third insole 30 with socks, the height of the inverted T-shaped projection part 31, that is, the heights of the vertical and lateral projection parts 32 and 33 is preferably 5mm or less.

The baby pinches the vertical projection part 32 by the first and second toes 1 and 2 even when wears socks. In this case, if the vertical project part 32 only exists, since the vertical projection 32 is low, the toes slip and cannot pinch it well. Since the lateral projection part 33 works so as to stop the slip of the toes, the pinching operation can be well performed.

Since the baby who just begun walking walks with flat soles, if the projection part extends to the bases of the fourth and fifth toes 4 and 5, the foot gets stuck with it at the time of walking, causing unbalanced walking. Therefore, the lateral projection part 33 preferably has one end which does not extend to the third toe 3.

As described above, the first insole 10 is used when the baby puts on shoes barefoot, and the third insole 30 is used when the baby puts on shoes with socks especially. Therefore, according to the insole set (Fig. 22) comprising the combination of the first insole 10 and the third insole 30, the formation of the arch of the foot of the baby who just begun walking can be promoted in both cases where the baby is barefoot and where the baby wears the socks.

There is a difference in height of the foot in the shoe by a thickness of the sock between the case where the baby is barefoot and the case the baby wears the socks. Thus, it is preferable that the fifth insole 50 having a flat configuration with no projection, shown in Fig. 16, is set in addition to the combination of the first and the third insoles 10 and 30 (Fig. 24). The fifth insole 50 is put on the first insole 10 when the baby puts on shoes barefoot. The fifth insole 50 is preferably put under the third insole 30 also when the third insole 30 is used when the baby is barefoot.

According to the second insole 20 shown in Fig. 9 and the fourth insole 40 shown in Fig. 14, each of them has a projection part for the arch of the foot abutting on the arch of the foot of the baby, in addition to the projection part between toes. The insoles 20 and 40 are suitable for the baby whose arch of the foot starts to form. When the arch of the foot starts to form, a track in the shift of the center of gravity draws an arc although the degree of its curvature is still small. Thus, when the insole having the projection part for the arch of the foot is used in this stage, since the projection exists at the arch of the foot, the shift of the center of gravity toward outside becomes larger. As a result, the degree of the arc-shaped curvature in the shift of the center of gravity is increased and the formation of the arch of the foot can be further promoted.

The second and fourth insoles 20 and 40 are described further in detail.

The second insole 20 comprises a partition wall 21 rising between the first and second toes 1 and 2 of the foot of the baby and also comprises a projection part 22 for the arch of the foot abutting on the arch of the foot of the baby. Since the partition wall 21 is substantially the same as the partition wall 11 of the first insole 10, its description is omitted. The projection part 22 for the arch of the foot projects so as to abut on a whole region of the arch of the foot of the baby as shown in Figs. 9 and 10.

The second insole 20 is used when the baby whose arch of the foot starts to form, puts on shoes barefoot. When the second insole 20 is used in this stage, the degree of the curvature of the track in the shift of the center of gravity is increased because of the projection 22 for the arch of the foot, in addition to the effect in which the movement of the toes can be promoted by the partition wall 21. As a result, the formation of the arch of the foot can be further promoted.

The insole set (Fig. 20) combining the first and second insoles 10 and 20 is used when the baby puts on shoes barefoot and can be used from the early stage of walking to the stage in which the arch of the foot starts to form.

The fourth insole 40 comprises an inverted T-shaped projection part 41 having a vertical projection part 42 projecting a little and extending between the first and second toes 1 and 2, and a lateral projection part 43 extending to the bases of the first and second toes 1 and 2, and also comprises a projection part 44 for the arch of the foot abutting on the arch of the foot of the baby. The inverted T-shaped projection part 41 is the substantially the same as the inverted T-shaped projection part 31 of the third insole 3, its description is omitted. The projection part 44 for the arch of the foot projects so as to abut on a whole region of the arch of the foot of the baby as shown in Figs. 14 and 15.

The fourth insole 40 is used when the baby whose arch of the foot starts to form, puts on shoes with socks. When the fourth insole 40 is used in this stage, the degree of the curvature of the track in the shift of the center of gravity is increased because of the projection 44 for the arch of the foot, in addition to the effect in which the movement of the toes can be promoted by the partition wall 41. As a result, the formation of the arch of the foot can be further promoted.

The insole set (Fig. 21) combining the third and fourth insoles 30 and 40 is used when the baby puts on shoes with socks and can be used from the early stage of walking to the stage in which the arch of the foot starts to form.

As described above, the second insole 20 is used when the baby puts on shoes barefoot, and the fourth insole 40 is used when the baby puts on shoes with socks especially. Therefore, according to the insole set (Fig. 23) comprising the combination of the second insole 20 and the fourth insole 40, the formation of the arch of the foot of the baby who just begun walking can be promoted in both cases where the baby is barefoot and where the baby wears the socks. Since there is a difference in height of the foot in the shoe by a thickness of the sock between the case where the baby is barefoot and the case where the baby wears the socks, it is preferable that the fifth insole 50 having a flat configuration with no projection, shown in Fig. 16, is set in addition to the combination of the second and the fourth insoles 20 and 40 (Fig. 25).

The insole set shown in Fig. 26 comprises the first insole 10, the second insole 20, the third insole 30, the fourth insole 40 and the fifth insole 50. The insole set can respond to both cases where the baby is barefoot and the baby wears the socks, and can be used from the early stage of walking to the stage in which the arch of the foot starts to form.

Although the present invention was described with reference to the drawings above, the present invention is not limited to the illustrated embodiments only. Various modifications and variations can be added in the same or an equivalent range of the present invention.

## Claims

1. An insole set for baby shoes comprising:
a first insole (10) having a partition wall (11) rising between the first and second toes of a baby foot; and
a second insole (30) having an inverted T-shaped projection part (31) comprising a vertical projection part (32) projecting a little and extending between the first and second toes and a lateral projection part (33) extending under bases of the first and second toes.

2. The insole set for baby shoes according to claim 1, further comprising a third insole (50) having a flat configuration with no projection.

3. The insole set for baby shoes according to claim 1 or 2, wherein a height of said partition wall (11) of the first insole (10) is the same as that of the toes of the baby foot or higher than the toes of the baby foot.

4. The insole set for baby shoes according to any one of claims 1 to 3, wherein a height of said vertical and lateral projections (32,33) of the second insole is 5mm or less.

5. The insole set for baby shoes according to claim 2, wherein said first insole (10) is for a case the baby is barefoot;
said second insole (30) is for a case the baby wears socks; and
said third insole (50) is for adjusting the height.

6. The insole set for baby shoes according to any one of claims 1 to 5, further comprising:
a fourth insole (20) having a partition wall (21) rising between the first and second toes of the baby foot and a projection (22) for an arch of the foot abutting on the arch of the baby foot; and
a fifth insole (40) having an inverted T-shaped projection part (41) comprising a vertical projection part (42) projecting a little and extending between the first and second toes and a lateral projection part (43) extending under bases of the first and second toes and a projection (44) for an arch of the foot abutting on the arch of the baby foot.
